# EUROPEAN PATENT APPLICATION

(11) **EP 4 537 806 A1**
(43) Date of publication of application: **16.04.2025**
(21) Application number: 23820072.9
(22) Date of filing: 05.06.2023
(51) Int. Cl.: A61K 8/34, A61K 8/37, A61K 8/9789, A61Q 19/00, A61Q 17/00

(54) **OLEOSOME PREPARATION COMPOSITION WITH EXCELLENT STABILITY AND ANTIMICROBIAL ACTIVITY COMPRISING BRANCHED DIOL-BASED COMPOUND AS ACTIVE INGREDIENT AND METHOD FOR PREPARING SAME**

(30) Priority: 07.06.2022 KR 20220068727
(71) Applicant: Samkyung Costech Co., Ltd, Suwon-si, Gyeonggi-do 16681 (KR)
(72) Inventor: KIM, Myung Kyoo, Yongin-si, Gyeonggi-do 17082 (KR); KIM, Hong Rip, Suwon-si, Gyeonggi-do 16295 (KR); KIM, Hye In, Suwon-si, Gyeonggi-do 16688 (KR)
(74) Representative: RGTH
(86) International application number: PCT/KR2023/007687
(87) International publication number: WO 2023/239136

(57) **Abstract**

The present invention relates to an oleosome preparation composition with excellent stability and antimicrobial activity, comprising a branched diol-based compound as an active ingredient and a method for preparing same. More specifically, by treating oleosomes with a mixture of a branched diol-based compound, a straight-chain diol-based compound, a glyceryl-based ether/ester compound, and other preservatives, the oleosomes have excellent formulation stability, exhibit excellent antimicrobial activity against bacteria, fungi, yeast, and the like, and have a preservative boosting effect.

## Description

### [Technical Field]

The present invention relates to an oleosome preparation composition with excellent stability and antimicrobial activity, containing a branched diol-based compound as an active ingredient and a method of preparing same. More specifically, the present invention relates to an oleosome preparation composition with excellent formulation stability and excellent antimicrobial contamination effect and a method of preparing the same.

### [Background Art]

Oleosomes are a certain inner structure of plant seeds, which store energy in the form of natural oil that has the function of delivering fragrance or functional substances to the skin without irritation. Oleosomes may be used as emulsifiers in various cosmetics, may be prepared into cold-pressed emulsions with low production cost, and have the ability to protect and release functional substances through a natural delivery method.

Triacylglycerol molecules in plant seeds are insoluble in aqueous environments and tend to be combined into oils. In order to store these insoluble triacylglycerols, plants have unique seed oil storage compartments with a diameter of about 1 to 10 µm, which are known variously as oil bodies, oleosomes, lipid bodies, and spherosomes (collectively called oleosomes) in plant seed cells (Huang, Ann. Rev. Plant Mol. Biol. 43: 177-200, 1992).

When vegetable oils are separated by a typical process that involves the use of organic solvents, the oleosome structure is destroyed, and a wet grinder and three-phase centrifuge must be used to obtain intact oleosomes (Guth PCT/US2009/002243).

However, oleosomes may be easily degraded when biological agents such as bacteria, fungi, and mycoplasma are exposed to fluid preparations. Preservatives may be added to protect the oleosomes from exposure to biological agents, and acid salts such as benzoates, salicylates, sorbates, and propionates, or acids such as dehydroacetic acid and ferulic acid are commonly used as preservatives.

The acid or acid salt preservative should be substantially present in its acid form, i.e., at a pH less than 6.0, preferably at a pH of 4.0 to 5.0 in order for the preservative to act as a preservative. However, in this acidic pH range of 4.0 to 5.0, the oleosome structure is weakened, thus causing oil leakage from the oleosome, causing the oleosome preparation to lose physical stability thereof, and eventually making mixing of the components to produce a final preparation containing intact oleosomes impossible. In addition, a pH range of 4.0 to 5.0, which is the isoelectric point range of oleosomes, makes the oleosome preparation unstable (Qi et al. 2017). At a lower acidic pH out of the isoelectric point, the oleosome preparation becomes stable, but is irritating to the skin for use as a cosmetic.

Oleosomes are most stable and do not cause oil leakage in neutral and alkaline pH ranges. Furthermore, the neutral pH range is most suitable in order for the oleosome preparations to be stable and non-irritating to the skin. However, in the neutral pH range, microbial growth easily occurs and microbial contamination reduces the stability of the oleosome preparation. Therefore, it is necessary to find an additive that can effectively maintain the stability of the oleosome preparation in the neutral pH range.

Generally, when oleosomes are treated with linear polyol compounds, glyceryl compounds and other preservatives, which are substances in the neutral pH range used in cosmetic compositions, the oleosome preparations become unstable and the physical properties are deteriorated. As a result of research on substances that can maintain the stability of oleosome preparations, the present inventors found that branched polyol compounds can maintain excellent oleosome stability without causing deterioration of oleosome preparations although used in combination with other compounds that destabilize the oleosome preparations. Accordingly, the present inventors developed a composition that effectively maintains the stability of oleosome preparations in a neutral pH range and has an effect of preventing microbial contamination that destroys oleosome preparations.

In addition, oleosomes are structures present in most plant seeds and thus it advantageously is easy to obtain oleosome raw materials and oleosome preparations. The plant seeds may include safflower seeds (*Carthamus tinctorius* seeds), hempseeds (*Cannabis sativa* seeds), sunflower seeds (*Helianthus annuus* seeds), coconut (*Cocos nucifera*), sesame seeds (*Torreya nucifera* seeds), camellia seeds (*Camellia japonica* seeds), green tea seeds (*Camellia sinensis* seeds), chia seeds (*Salvia hispanica* seeds), pomegranate seeds *(Punica granatum* seeds), moringa seeds *(Moringa oleifera* seeds), tea oil seeds (*Camellia oleifera* seeds), rapeseed (*Brassica napus* seeds), yuzu seeds (*Citrus junos* seeds), olives (*Olea europaea*), grape seeds (*Vitis vinifera* seeds), soybeans (*Glycine max*), rose hips (*Rosa canina*), macadamia nuts (Macadamia), meadowfoam seeds (*Limnanthes alba* seeds), borage (*Borago officinalis*)*,* cotton seeds (*Gossypium hirstutum*), rice bran (*Oryza Sativa* seeds), apricot kernel *(Prunus armeniaca* seeds), corn (*Zea mays*), horse chestnut fruits (*Aesculus hippocastanum* fruits), palm (*Elaeis Guineensis*), palm kernel (Elaeis Guineensis Kernel), castor bean (*Ricinus communis*), hazelnut (*Corylus heterophylla*), jojoba (*Simmondsia chinensis*), avocado (*Persea americana*), walnut (*Juglans regia* seeds), peanut (*Arachis Hypogaea*), argan (*Argania Spinosa*), and the like.

Meanwhile, safflower (*Carthamus tinctorius* L.) is an annual plant belonging to the Asteraceae family, is native to the mountainous regions of Afghanistan or Ethiopia, and grows in China, Tibet, or the like. The medicinal parts of safflower include flowers and seeds, and safflower has effects of inhibiting platelet coagulation, delaying bleeding time, and lowering plasma cholesterol and neutral fat. In particular, safflower seed oil contains a large amount of unsaturated fatty acids including linoleic acid and tocopherol, and is used as a new material with antioxidant, antithrombotic, and antihypertensive activities. For this purpose, roasted safflower seeds are extracted and used as edible oil.

Meanwhile, sunflower seeds, which are seeds of sunflower (*Helianthus annuus*), contain about 50% oil, which includes linolenic acid, phospholipids, beta-sitosterol, glycolipids, ceramides, or the like. The sugars contained in the phosphorus range from monosaccharides to trisaccharides, and contain organic acids such as citric acid and tartaric acid, as well as beta-carotene. The protein constituting sunflower seeds contain lots of essential amino acids, especially arginine. Phospholipids, beta-sitosterol, glycolipids, and ceramides are lipid membrane components of the keratinocyte layer that maintain moisture loss at an appropriate level, forming a skin barrier and providing excellent skin-moisturizing and softening effects.

Meanwhile, hempseed oil is obtained by cold pressing the cannabis tree, contains essential fatty acids, vitamins A, D, and E, minerals, omega 3, and omega 6, and is the only oil that supplies omega 3 and omega 6, excluding fish oil. It contains more essential fatty acids than other vegetable oils, and contains vitamin E, a natural antioxidant, and sterols that block the absorption of cholesterol. In addition, hempseed oil is rich in alpha-linolenic acid (ALA) and gamma-linolenic acid, is highly effective in moisturizing and softening the skin, and contains abundant minerals such as phosphorus, potassium, magnesium, sulfur, calcium, iron, and zinc. Hempseed oil lowers cholesterol levels, treats vascular inflammation, and purifies the blood to reduce high blood pressure, heart disease, and stroke. In particular, the hempseed oil is highly effective in moisturizing, antiaging, and regenerating when used on the skin and is known to relieve itching when used on the atopic skin.

Meanwhile, rapeseed (*Brassica napus* L.) is a biennial plant belonging to the Papaveraceae family of dicotyledonous plants, and is mainly grown on the southern islands of Korea and Jeju Island. Rapeseed (*Brassica napus* L.) was cultivated for ornamental purposes, but has recently become a major oil crop cultivated to produce edible oil and biodiesel. Rapeseed contains 35 to 45% oil, and is widely used as cosmetics for moisturizing the skin, biofuels, or the like. in addition to edible oil due to a high content of oleic acid, a typical unsaturated fatty acid.

Meanwhile, camellia (*Camellia japonica*) is an evergreen tree native to southern Korea, Japan, and China, mainly grows in mountainous areas, coastal areas, and near villages, and is distributed in the southern part of the Korean peninsula. Camellia oil is a fatty oil obtained by drying and crushing the seeds of Camellia japonica L. and its relatives in the Theaceae family, and then squeezing or extracting with a solvent. It contains more than 80% of oleic acid with respect to the total content of fatty acid. Oleic acid is the main component of fatty acids contained in olive oil, or the like, and is an omega-9 unsaturated fatty acid.

Camellia oil does not contain any cholesterol, and contains vitamin E, omega-6, omega-9, the phenolic compounds tyrosol and hydroxytyrosol, and the glycosides oleuropein and ligstroside, and thus has excellent antioxidant activity. Camellia oil is utilized in creams, emulsions, or the like for similar applications to olive oil, and is especially widely used in hair products owing to the excellent conditioning effect thereof. In addition, Camellia oil is effective in treating atopic dermatitis because it is not irritating to the skin, has good absorbency and has an excellent moisturizing effect. It is suitable as a base oil for massage because it does not deteriorate easily and is well absorbed into the skin. In addition, it has the effect of inhibiting sebum oxidation and is widely used for aging skin and dry skin.

Meanwhile, yuzu (*Citrus junos*) originated in China and was introduced to Korea. The seeds and peel of yuzu contain limonene and limonoids, which have a bitter taste. Among the essential oil components, limonene has blood pressure lowering, antioxidant and antibacterial effects, limonoid has anticancer effects, and phenol and flavonoid have antioxidant effects. Therefore, yuzu (*Citrus junos*) is used as a folk remedy for detoxification, relieving pain, and colds. Yuzu seeds are rich in natural fragrance and essential oil, and yuzu flesh contains amino acids, free sugars, and organic acids. Despite containing beneficial essential oil components, yuzu seeds have not been actively used until now. More than 1,800 tons of Citrus Junos seeds are produced annually. There is a need to expand the scope of the application thereof using various methods.

### [Disclosure]

### [Technical Problem]

Therefore, the present invention has been made in view of the above problems, and it is an object of the present invention to provide a method of preparing an oleosome preparation having excellent formulation stability and antimicrobial contamination effects, and an oleosome preparation produced therefrom.

### [Technical Solution]

In accordance with an aspect of the present invention, the above and other objects can be accomplished by the provision of a method of preparing an oleosome preparation composition having excellent stability and antibacterial properties including 1) preparing an oleosome, 2) treating the oleosome with a mixture of a C1 to C5 branched diol compound, a C1 to C10 linear diol compound, and a glyceryl ether/ester compound or other preservative, and 3) preparing an oleosome having a pH of 5.0 to 8.0.

The branched diol compound may include at least one selected from the group consisting of 2-methyl-1,3-propanediol, 2,2-dimethyl-1,3-propanediol, 2-methyl-2-propyl-1,3-propanediol, and 3-methyl-1,3-butanediol. Preferably, the branched diol compound may be 2-methyl-1,3-propanediol.

The linear diol compound may include at least one selected from the group consisting of 1,3-propanediol, 1,3-butanediol, 1,2-hexanediol, and 1,2-octanediol. Preferably, the linear diol compound may be 1,2-hexanediol.

The glyceryl ether/ester compound may include at least one selected from the group consisting of ethylhexylglycerin, glyceryl caprylate, glyceryl stearate, glyceryl undecylenate, caprylyl glyceryl ether, and glyceryl laurate. Preferably, the glyceryl ether compound may be ethylhexylglycerin, and the glyceryl ester compound may be glyceryl caprylate.

Preferably, the other preservative may be phenoxyethanol.

Step 1) may include soaking seeds in water, washing the seeds, wet-grinding the washed seeds with water at a weight ratio of 1:2, filtering the resulting seeds to obtain a ground solution, mixing the ground solution with sodium bicarbonate, and centrifuging the resulting mixture to obtain oleosomes.

The seeds may include safflower seeds (*Carthamus tinctorius* seeds), hempseeds (*Cannabis sativa* seeds), sunflower seeds (*Helianthus annuus* seeds), coconut (*Cocos nucifera*), sesame seeds (*Torreya nucifera* seeds), camellia seeds (*Camellia japonica* seeds), green tea seeds (*Camellia sinensis* seeds), chia seeds (*Salvia hispanica* seeds), pomegranate seeds (*Punica granatum* seeds), moringa seeds (*Moringa oleifera* seeds), tea oil seeds (*Camellia oleifera* seeds), rapeseed (*Brassica napus* seeds), yuzu seeds (*Citrus junos* seeds), olives (*Olea europaea*), grape seeds (*Vitis vinifera* seeds), soybeans (*Glycine max*), rose hips (*Rosa canina*), macadamia nuts (Macadamia), meadowfoam seeds (*Limnanthes alba* seeds), borage (*Borago officinalis*), cotton seeds (*Gossypium hirstutum*), rice bran (*Oryza Sativa* seeds), apricot kernel (*Prunus armeniaca* seeds), corn (*Zea mays*), horse chestnut fruits (*Aesculus hippocastanum* fruits), palm (*Elaeis Guineensis*), palm kernel (Elaeis Guineensis Kernel), castor bean (*Ricinus communis*)*,* hazelnut (*Corylus heterophylla*)*,* jojoba (*Simmondsia chinensis*)*,* avocado (*Persea americana*), walnut (*Juglans regia* seeds), peanut (*Arachis Hypogaea*), or argan (*Argania Spinosa*).

In accordance with another aspect of the present invention, there are provided an oleosome preparation composition having excellent stability and antibacterial properties prepared using the method, and a cosmetic product containing the oleosome preparation composition.

The cosmetic product may be selected from the group consisting of skin care products, makeup products, hair care products, sunscreens, body care products, hand sanitizers, deodorants, exfoliating products, topical products, dermatological products, and acne care products.

In accordance with another aspect of the present invention, there is an oleosome preparation composition having excellent stability and antibacterial properties containing 79.3 to 94.5 parts by weight of oleosome, 0.5 to 4.0 parts by weight of 1,2-hexanediol, 0.01 to 0.1 parts by weight of ethylhexylglycerin, 0.1 to 1 part by weight of glyceryl caprylate, and 4.8 to 20 weight parts of 2-methyl-1,3-propanediol.

The oleosome preparation composition may have a pH of 5.0 to 8.0.

The oleosome preparation composition may be formulated as a pack, an ointment, a lotion, a solubilization, a suspension, an emulsion, a cream, a gel, a spray, a papule, a plaster, a patch, or a pain relief patch.

### [Advantageous effects]

The oleosome preparation composition prepared using the preparation method according to the present invention exbibits excellent stability in the neutral pH range by treating the oleosome with a branched diol compound. In addition, the oleosome preparation composition has the effect of overcoming the side effect of breaking the oleosome preparation and greatly improving the stability of the oleosome preparation using the branched diol compound in a combination of a linear diol compound, a glyceryl ether/ester compound, and other preservatives that break the oleosome preparation. In addition, it inhibits the growth of bacteria, fungi, or the like, prevents microbial contamination and has an excellent preservative effect and a superior preservative boosting effect, thus being useful for cosmetics.

In addition, an oleosome preparation composition can be prepared in an environmentally friendly manner without an emulsifier or solvent by preparing oleosomes having a formulation of an oil-in-water emulsion from seeds.

### [Description of Drawings]

FIG. 1 shows the results of stability test of the oleosome preparation composition from each mixture according to one embodiment of the present invention.
FIG. 2 shows the results of stability test of the oleosome preparation composition from each mixture according to one embodiment of the present invention.
FIG. 3 shows the results of stability test of the oleosome preparation composition depending on pH according to one embodiment of the present invention.
FIG. 4 shows the results of the test of the antimicrobial contamination effect according to one embodiment of the present invention.
FIG. 5 shows the results of the test of the antimicrobial contamination effect of glyceryl caprylate alone according to one embodiment of the present invention.
FIG. 6 shows the result of the test of the antimicrobial contamination effect of a mixture of HD 1% and MPD 5% according to one embodiment of the present invention.

### [Best Mode]

Hereinafter, the present invention will be described in more detail.

The term "oleosome" herein refers to any discrete subcellular oil or wax storage organelle that may be obtained from living cells. Oleosomes may be obtained from any cell containing the organelle, including plant cells, fungal cells, yeast cells (Leber, R et al, 1994, Yeast 10: 1421-28), bacterial cells (Pieper-Fuerst et al., 1994, J Bacteriol. 176: 4328-37), and algal cells (Roessler, PG, 1988, J Phycol. (London) 24: 394-400).

In a preferred embodiment of the present invention, the oleosome is obtained from plant cells, wherein the cell includes cells of pollen, spores, seeds and vegetative plant organs, each of which contains the oleosome [General example: Huang, Ann Rev Plant Physiol. 43: 177-200 (1992)]. More preferably, the oleosome used herein is obtained from a plant seed.

The oleosome contains an oil-in-water emulsion formed by oily globules provided with a lamellar liquid crystal coating dispersed in an aqueous phase.

In this invention, oleosome may be a fluid produced from safflower (*Carthamus tinctorius*) seeds, but is not limited thereto, and may, for example, be a fluid produced from hempseeds (*Cannabis sativa* seeds), sunflower seeds (*Helianthus annuus* seeds), coconut (*Cocos nucifera*), sesame seeds (*Torreya nucifera* seeds), camellia seeds (*Camellia japonica* seeds), green tea seeds (*Camellia sinensis* seeds), chia seeds (*Salvia hispanica* seeds), pomegranate seeds (*Punica granatum* seeds), moringa seeds *(Moringa oleifera* seeds), tea oil seeds (*Camellia oleifera* seeds), rapeseed (*Brassica napus* seeds), yuzu seeds (*Citrus junos* seeds), olives (*Olea europaea*), grape seeds (*Vitis vinifera* seeds), soybeans (*Glycine max*), rose hips (*Rosa canina*)*,* macadamia nuts (Macadamia), meadowfoam seeds (*Limnanthes alba* seeds), borage (*Borago officinalis*)*,* cotton seeds (*Gossypium hirstutum*), rice bran (*Oryza Sativa* seeds), apricot kernel (*Prunus armeniaca* seeds), corn (*Zea mays*), horse chestnut fruits (*Aesculus hippocastanum* fruits), palm (*Elaeis Guineensis*), palm kernel (Elaeis Guineensis Kernel), castor bean (*Ricinus communis*), hazelnut (*Corylus heterophylla*)*,* jojoba (*Simmondsia chinensis*), avocado (*Persea americana*), walnut (*Juglans regia* seeds), peanut (*Arachis Hypogaea*), argan (*Argania Spinosa*), and the like.

The term "diol" herein refers to a compound containing two hydroxyl groups (-OH).

The present invention provides a method of preparing an oleosome preparation composition having excellent stability and antibacterial properties and an oleosome preparation composition prepared therefrom.

The method of preparing an oleosome preparation composition having excellent stability and antibacterial properties according to the present invention includes 1) preparing an oleosome, 2) treating the oleosome with a mixture of a C1 to C5 branched diol, a C1 to C10 linear diol, and a glyceryl ether/ester or other preservative, and 3) preparing an oleosome at a pH of 5.0 to 8.0.

The antibacterial activity refers to an activity inhibitory effect against bacteria, fungi, or viruses.

The bacteria include, but are not limited to, *Bacillus subtilis, Pseudomonas aeruginosa, Escherichia coli,* or *Staphylococcus aureus.*

The fungus may include, but is not limited to, *Candida albicans* or *Aspergillus niger.*

Step 1) includes soaking the seeds in water, washing the seeds, wet-grinding the washed seeds with water at a weight ratio of 1:2, filtering the result to obtain a ground solution, mixing the ground solution with sodium bicarbonate, and centrifuging the resulting mixture to obtain oleosomes.

The seeds may include, but is not limited to safflower seeds (*Carthamus tinctorius* seeds), hempseeds (*Cannabis sativa* seeds), sunflower seeds (*Helianthus annuus* seeds), coconut (*Cocos nucifera*), sesame seeds (*Torreya nucifera* seeds), camellia seeds (*Camellia japonica* seeds), green tea seeds (*Camellia sinensis* seeds), chia seeds (*Salvia hispanica* seeds), pomegranate seeds (*Punica granatum* seeds), moringa seeds (*Moringa oleifera* seeds), tea oil seeds (*Camellia oleifera* seeds), rapeseed (*Brassica napus* seeds), yuzu seeds (*Citrus junos* seeds), olives (*Olea europaea*), grape seeds (*Vitis vinifera* seeds), soybeans (*Glycine max*), rose hips (*Rosa canina*), macadamia nuts (Macadamia), meadowfoam seeds (*Limnanthes alba* seeds), borage (*Borago officinalis*), cotton seeds (*Gossypium hirstutum*), rice bran (*Oryza Sativa* seeds), apricot kernel (*Prunus armeniaca* seeds), corn (*Zea mays*), horse chestnut fruits (*Aesculus hippocastanum* fruits), palm (*Elaeis Guineensis*), palm kernel (Elaeis Guineensis Kernel), castor bean (*Ricinus communis*), hazelnut (*Corylus heterophylla*)*,* jojoba (*Simmondsia chinensis*), avocado (*Persea americana*), walnut (*Juglans regia* seeds), peanut (*Arachis Hypogaea*), argan (*Argania Spinosa*), and the like.

In the step of soaking the seeds in water, the soaking may be performed for 1 to 2 days, but is not limited thereto. Prior to grinding, the seeds may be soaked in water to allow the seeds to absorb the liquid, thereby softening the cell walls, and facilitating the grinding process. Long-term absorption may mimic a germination process. Therefore, the composition of the seed components may be changed to beneficial levels.

In addition, the ground seed fraction may be filtered to remove solid contaminants such as seed hulls, fibrous materials, insoluble carbohydrates and proteins, and other insoluble contaminants, and the filtration may include, but is not limited to, mesh-based primary filtration and cotton cloth-based secondary filtration.

The concentration of the sodium bicarbonate may be 0.1 N, but is not limited thereto.

The branched diol compound may include at least one selected from the group consisting of 2-methyl-1,3-propanediol, 2,2-dimethyl-1,3-propanediol, 2-methyl-2-propyl-1,3-propanediol, and 3-methyl-1,3-butanediol, and preferably 2-methyl-1,3-propanediol.

The linear diol compound may be selected from the group consisting of 1,3-propanediol, 1,3-butanediol, 1,2-hexanediol, and 1,2-octanediol, and preferably 1,2-hexanediol.

The glyceryl ether/ester compound may be selected from the group consisting of ethylhexylglycerin, glyceryl caprylate, glyceryl stearate, glyceryl undecylenate, caprylyl glyceryl ether, and glyceryl laurate, preferably ethylhexylglycerin and/or glyceryl caprylate.

The other preservative may be phenoxyethanol.

Step 2) may preferably be treating the oleosome with a mixture of 2-methyl-1,3-propanediol, 1,2-hexanediol, ethylhexylglycerin, glyceryl caprylate, and phenoxyethanol.

When the branched diol compound and the linear diol compound are used in combination, they have a better antimicrobial contamination effect than when used alone, and have an effect of boosting the preservative activity of compounds that have insufficient preservative effects when used alone. In addition, the branched diol compound functions to prevent the linear diol compound, glyceryl ether/ester compound, or other preservatives from breaking down the oleosome preparation, and to stably maintain the oleosome preparation.

In addition, in another aspect, the present invention provides an oleosome preparation composition having excellent stability and antibacterial properties prepared using the preparation method and a cosmetic product containing the same.

The cosmetic product may be selected from the group consisting of skin care products, makeup products, hair care products, sunscreens, body care products, hand sanitizers, deodorants, exfoliating products, topical products, dermatological products, and acne care products, but is not limited thereto.

In addition, the present invention provides an oleosome preparation composition with excellent stability and antibacterial properties, which contains 79.3 to 94.5 parts by weight of oleosome, 0.5 to 4.0 parts by weight of 1,2-hexanediol, 0.01 to 0.1 parts by weight of ethylhexylglycerin, 0.1 to 1 part by weight of glyceryl caprylate, and 4.8 to 20 parts by weight of 2-methyl-1,3-propanediol.

The pH of the composition is 5.0 to 8.0 and the stability of the oleosome preparation composition is maintained within the pH range.

There is no limitation as to the type of the formulation of the oleosome preparation composition prepared by mixing the ingredients within the ranges and the oleosome preparation composition may be incorporated into any formulation known in the art. Examples of the formulation include cosmetics such as lotions, creams, and emulsions, as well as external skin preparations such as ointments, solubilizations, suspensions, gels, sprays, plasters, papules, patches, and pain relief patches. The mix amount of each of these formulations may be appropriately selected and mixed using techniques conventional in the art to achieve the desired effects and, for example, the oleosome composition may be contained in an amount of 0.1 wt% to 1.0 wt% based on the total weight of the cosmetic composition, but is not limited thereto.

Hereinafter, the present invention will be described in detail with reference to examples, but the present invention is not limited to these examples.

### Example 1. Preparation of oleosome

1 kg of plant seeds was soaked in 1 L of water for 1 to 2 days at room temperature and then washed with water 1 to 2 times. The washed seeds were wet-ground with water in a ratio of 1:2 using a millstone grinder, and were primarily filtered through a mesh net and secondly filtered through a cotton cloth to obtain a ground solution. Water and sodium bicarbonate were supplied in a final volume of 7 L so that the sodium bicarbonate concentration was 0.1 N and then the solution was centrifuged using a three-phase centrifuge at room temperature to obtain oleosomes.

An oleosome composition of each plant seed was prepared using the preparation method of Example 1. In Examples 1-1 to 1-36 below, the linear diol compound is 1,3-propanediol (hereinafter abbreviated as "PD"), 1,3-butanediol (hereinafter abbreviated as "BD"), 1,2-hexanediol (hereinafter abbreviated as "HD") or 1,2-octanediol (hereinafter abbreviated as "OD"), the branched diol compound is 2-methyl-1,3-propanediol (hereinafter abbreviated as "MP"), 2,2-dimethyl-1,3-propanediol (hereinafter abbreviated as "DP"), 2-methyl-2-propyl-1,3-propanediol (hereinafter abbreviated as "MPP) or 3-methyl-1,3-butanediol (hereinafter abbreviated as MB), the glyceryl ether compound is ethylhexylglycerin (hereinafter abbreviated as "EHG"), the glyceryl ester compound is glyceryl caprylate (hereinafter abbreviated as "GC"), and another preservative is phenoxyethanol (hereinafter abbreviated as "PE").

### [Example 1-1] to [Example 1-11]

### Safflower oleosome

A safflower oleosome composition was prepared from safflower seeds as plant seeds using the preparation method of Example 1. Table 1 below shows the components and contents (parts by weight) of the safflower oleosome composition.

**[Table 1]**

| | Safflower oleosome | PD | BD | HD | OD | MP | DP | MPP | MB | EHG | PE | GC |
|---|---|---|---|---|---|---|---|---|---|---|---|---|
| Example 1-1 | 90 | | | | | 10 | | | | | | |
| Example 1-2 | 90 | | | | | | 10 | | | | | |
| Example 1-3 | 90 | | | | | | | 10 | | | | |
| Example 1-4 | 90 | | | | | | | | 10 | | | |
| Example 1-5 | 88 | | | 2 | | 10 | | | | | | |
| Example 1-6 | 88 | | | 2 | | | 10 | | | | | |
| Example 1-7 | 88 | | | 2 | | | | 10 | | | | |
| Example 1-8 | 88 | | | 2 | | | | | 10 | | | |
| Example 1-9 | 93.95 | | | 1 | | 5 | | | | 0.05 | | |
| Example 1-10 | 93 | | | 1 | | 5 | | | | | 1 | |
| Example 1-11 | 93.5 | | | 1 | | 5 | | | | | | 0.5 |

### [Example 1-12] to [Example 1-22]

### Hempseed oleosome

A hempseed oleosome composition was prepared from hempseed seeds as plant seeds using the preparation method of Example 1. Table 2 below shows the components and contents (parts by weight) of the hempseed oleosome composition.

**[Table 2]**

| | Hempseed Oleosome | PD | BD | HD | OD | MP | DP | MPP | MB | EHG | PE | GC |
|---|---|---|---|---|---|---|---|---|---|---|---|---|
| Example 1-12 | 90 | | | | | 10 | | | | | | |
| Example 1-13 | 90 | | | | | | 10 | | | | | |
| Example 1-14 | 90 | | | | | | | 10 | | | | |
| Example 1-15 | 90 | | | | | | | | 10 | | | |
| Example 1-16 | 88 | | | 2 | | 10 | | | | | | |
| Example 1-17 | 88 | | | 2 | | | 10 | | | | | |
| Example 1-18 | 88 | | | 2 | | | | 10 | | | | |
| Example 1-19 | 88 | | | 2 | | | | | 10 | | | |
| Example 1-20 | 93.95 | | | 1 | | 5 | | | | 0.05 | | |
| Example 1-21 | 93 | | | 1 | | 5 | | | | | 1 | |
| Example 1-22 | 93.5 | | | 1 | | 5 | | | | | | 0.5 |

### [Example 1-23] to [Example 1-36]

### Camellia oleosome

A camellia oleosome composition was prepared from camellia seeds as plant seeds using the preparation method of Example 1. Table 3 below shows the components and contents (weight parts) of the camellia oleosome composition.

**[Table 3]**

| | Camellia oleosome | PD | BD | HD | OD | MP | DP | MPP | MB | EHG | PE | GC |
|---|---|---|---|---|---|---|---|---|---|---|---|---|
| Example 1-23 | 90 | | | | | 10 | | | | | | |
| Example 1-24 | 90 | | | | | | 10 | | | | | |
| Example 1-25 | 90 | | | | | | | 10 | | | | |
| Example 1-26 | 90 | | | | | | | | 10 | | | |
| Example 1-27 | 88 | | | 2 | | 10 | | | | | | |
| Example 1-28 | 88 | | | 2 | | | 10 | | | | | |
| Example 1-29 | 88 | | | 2 | | | | 10 | | | | |
| Example 1-30 | 88 | | | 2 | | | | | 10 | | | |
| Example 1-31 | 95 | | | | | 5 | | | | | | |
| Example 1-32 | 94 | | | 1 | | 5 | | | | | | |
| Example 1-33 | 97 | | | 0.5 | | 2.5 | | | | | | |
| Example 1-34 | 93.95 | | | 1 | | 5 | | | | 0.05 | | |
| Example 1-35 | 93 | | | 1 | | 5 | | | | | 1 | |
| Example 1-36 | 93.5 | | | 1 | | 5 | | | | | | 0.5 |

### [Comparative Example]

Oleosomes were prepared in the same manner as in Example 1, but Comparative Compositions were prepared as safflower oleosome compositions (Comparative Examples 1-1 to 1-5), hempseed oleosome compositions (Comparative Examples 1-6 to 1-10), and camellia oleosome compositions (Comparative Examples 1-11 to 1-25) with reference to Tables 4, 5, and 6 below.

**[Table 4]**

| | Safflower oleosome | PD | BD | HD | OD | MP | DP | MPP | MB | EHG | PE | GC |
|---|---|---|---|---|---|---|---|---|---|---|---|---|
| Comparat ive Example 1-1 | 100 | | | | | | | | | | | |
| Comparat ive Example 1-2 | 90 | 10 | | | | | | | | | | |
| Comparat ive Example 1-3 | 90 | | 10 | | | | | | | | | |
| Comparat ive Example 1-4 | 90 | | | 10 | | | | | | | | |
| Comparat ive Example 1-5 | 90 | | | | 10 | | | | | | | |

**[Table 5]**

| | Hempseed oleosome | PD | BD | HD | OD | MP | DP | MPP | MB | EHG | PE | GC |
|---|---|---|---|---|---|---|---|---|---|---|---|---|
| Comparati ve Example 1-6 | 100 | | | | | | | | | | | |
| Comparati ve Example 1-7 | 90 | 10 | | | | | | | | | | |
| Comparati ve Example 1-8 | 90 | | 10 | | | | | | | | | |
| Comparati ve Example 1-9 | 90 | | | 10 | | | | | | | | |
| Comparati ve Example 1-10 | 90 | | | | 10 | | | | | | | |

**[Table 6]**

| | Camellia oleosome | PD | BD | HD | OD | MP | DP | MPP | MB | EHG | PE | GC |
|---|---|---|---|---|---|---|---|---|---|---|---|---|
| Comparati ve Example 1-11 | 100 | | | | | | | | | | | |
| Comparati ve Example 1-12 | 90 | 10 | | | | | | | | | | |
| Comparati ve Example 1-13 | 90 | | 10 | | | | | | | | | |
| Comparati ve Example 1-14 | 90 | | | 10 | | | | | | | | |
| Comparati ve Example 1-15 | 90 | | | | 10 | | | | | | | |
| Comparati ve Example 1-16 | 98 | | | 2 | | | | | | | | |
| Comparati ve Example 1-17 | 99 | | | 1 | | | | | | | | |
| Comparati ve Example 1-18 | 99.5 | | | 0.5 | | | | | | | | |
| Comparati ve Example 1-19 | 99.7 | | | | | | | | | 0.3 | | |
| Comparati ve Example 1-20 | 99.5 | | | | | | | | | 0.5 | | |
| Comparati ve Example 1-21 | 99 | | | | | | | | | | 1 | |
| Comparati ve Example 1-22 | 98 | | | | | | | | | | 2 | |
| Comparati ve Example 1-23 | 99.5 | | | | | | | | | | | 0.5 |
| Comparati ve Example 1-24 | 99 | | | | | | | | | | | 1 |
| Comparati ve Example 1-25 | 98.5 | | | | | | | | | | | 1.5 |

### Experimental Example 1. Evaluation of stability of oleosome preparation compositions obtained depending on mixing of branched diol compound, linear diol compound, glyceryl ether/ester compound, and other preservative compound

Oleosomes were treated with a branched diol compound, a linear diol compound, a glyceryl ether/ester compound, and other preservative compound in the pH range of 5 to 8 where oleosome preparations are stable, and centrifuged at 10,000 rpm and the stability of the oleosome preparation composition was determined. The degree of oil separation from oleosomes was determined over time and the degree of separation was visually evaluated and indicated as +++ (very stable), ++ (stable), + (slightly stable), - (slightly unstable),-(unstable), and --- (very unstable).

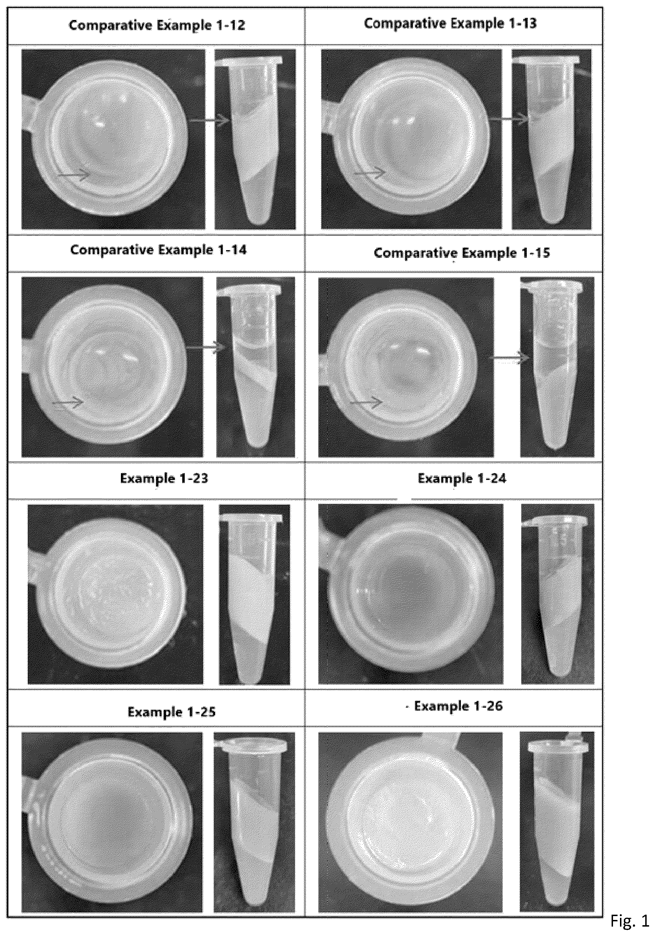

As can be seen from Table 7 and FIG. 1 below, when oleosome preparations are treated with linear diol compounds, glyceryl ether/ester compounds, and other preservatives alone, such as in Comparative Examples 1-1 to 1-15, 1-20, 1-22, and 1-25, the oleosome preparations became unstable and oil separation occurred. However, when oleosome preparations are treated with branched C1 to C5 diol compounds alone, such as in Examples 1-1 to 1-4, 1-12 to 1-15, and 1-23 to 1-26, the oleosome preparations were much more stable compared to linear diol compounds, glyceryl ether/ester compounds, and other preservatives, and oil separation did not occur.

**[Table 7]**

| Composition | Centrifugation time | | | |
|---|---|---|---|---|
| | 30 sec | 3 min | 5 min | 10 min |
| Comparative Example 1-1 | +++ | +++ | +++ | +++ |
| Comparative Example 1-2 | + | - | - | - |
| Comparative Example 1-3 | + | - | - | - |
| Comparative Example 1-4 | + | + | -- | --- |
| Comparative Example 1-5 | --- | --- | --- | --- |
| Comparative Example 1-6 | ++ | ++ | ++ | ++ |
| Comparative Example 1-7 | + | -- | -- | -- |
| Comparative Example 1-8 | + | -- | -- | -- |
| Comparative Example 1-9 | -- | ---- | --- | --- |
| Comparative Example 1-10 | --- | --- | --- | --- |
| Comparative Example 1-11 | +++ | +++ | +++ | +++ |
| Comparative Example 1-12 | + | - | - | -- |
| Comparative Example 1-13 | + | - | - | -- |
| Comparative Example 1-14 | -- | - | --- | --- |
| Comparative Example 1-15 | -- | --- | --- | --- |
| Comparative Example 1-20 | + | --- | --- | - |
| Comparative Example 1-22 | + | - | - | -- |
| Comparative Example 1-25 | + | + | - | - |
| Example 1-1 | +++ | +++ | +++ | +++ |
| Example 1-2 | +++ | +++ | +++ | +++ |
| Example 1-3 | +++ | +++ | +++ | +++ |
| Example 1-4 | +++ | +++ | +++ | ++ |
| Example 1-12 | +++ | +++ | +++ | +++ |
| Example 1-13 | +++ | +++ | +++ | ++ |
| Example 1-14 | +++ | +++ | ++ | ++ |
| Example 1-15 | +++ | +++ | ++ | ++ |
| Example 1-23 | +++ | +++ | +++ | +++ |
| Example 1-24 | +++ | +++ | +++ | +++ |
| Example 1-25 | +++ | +++ | +++ | ++ |
| Example 1-26 | +++ | +++ | ++ | ++ |

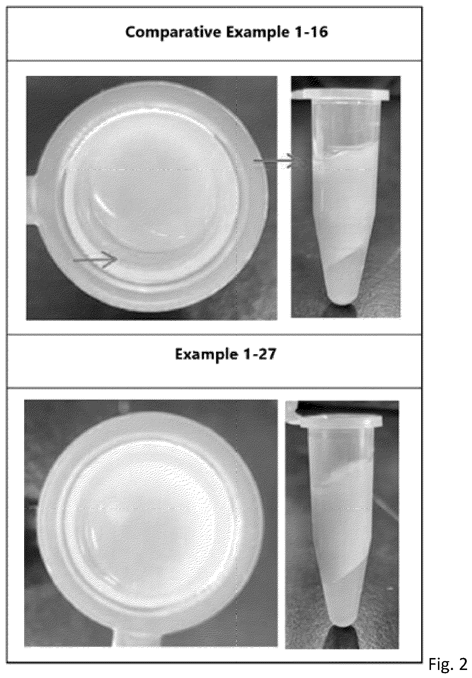

In addition, as can be seen from Table 8 and FIG. 2 below, when oleosomes were treated with a dispersion of a mixture of a linear diol compound and a glyceryl ether/ester compound or other preservative in a branched diol compound, the preparation was stably maintained without being broken (Examples 1-5 to 1-11, 1-16 to 1-22, 1-27 to 1-30, 1-34 to 1-36), which indicates that the branched diol compound stably maintains the oleosome preparation.

**[Table 8]**

| Composition | Centrifugation time | | | |
|---|---|---|---|---|
| | 30 sec | 3 min | 5 min | 10 min |
| Comparative Example 1-16 | + | - | -- | -- |
| Example 1-5 | +++ | +++ | +++ | +++ |
| Example 1-6 | +++ | +++ | +++ | +++ |
| Example 1-7 | +++ | +++ | +++ | +++ |
| Example 1-8 | +++ | +++ | +++ | +++ |
| Example 1-9 | +++ | +++ | +++ | +++ |
| Example 1-10 | +++ | +++ | +++ | +++ |
| Example 1-11 | +++ | +++ | ++ | ++ |
| Example 1-16 | +++ | +++ | +++ | +++ |
| Example 1-17 | +++ | ++ | ++ | ++ |
| Example 1-18 | +++ | ++ | ++ | ++ |
| Example 1-19 | +++ | ++ | ++ | ++ |
| Example 1-20 | +++ | ++ | ++ | ++ |
| Example 1-21 | +++ | ++ | ++ | ++ |
| Example 1-22 | +++ | ++ | ++ | ++ |
| Example 1-27 | +++ | +++ | +++ | +++ |
| Example 1-28 | +++ | +++ | +++ | +++ |
| Example 1-29 | +++ | +++ | +++ | +++ |
| Example 1-30 | +++ | +++ | +++ | +++ |
| Example 1-34 | +++ | +++ | +++ | +++ |
| Example 1-35 | +++ | +++ | +++ | +++ |
| Example 1-36 | +++ | +++ | +++ | +++ |

### Experimental Example 2. Determination of optimal pH for stability of oleosome preparation composition

The environments of oleosomes were adjusted to acidic, neutral, and alkaline conditions and then a formulation stability test was conducted. The pH was adjusted to these three conditions and the oleosomes were treated with a mixture of 1,2-hexanediol (hereinafter abbreviated as HD) and 2-methyl-1,3-propanediol (hereinafter abbreviated as MPD), the degree of oil separation from the oleosomes was observed over time when centrifugation was performed at 10,000 rpm, and the degree of separation was visually evaluated and indicated as +++ (very stable), ++ (stable), + (slightly stable), - (slightly unstable),-(unstable), and --- (very unstable).

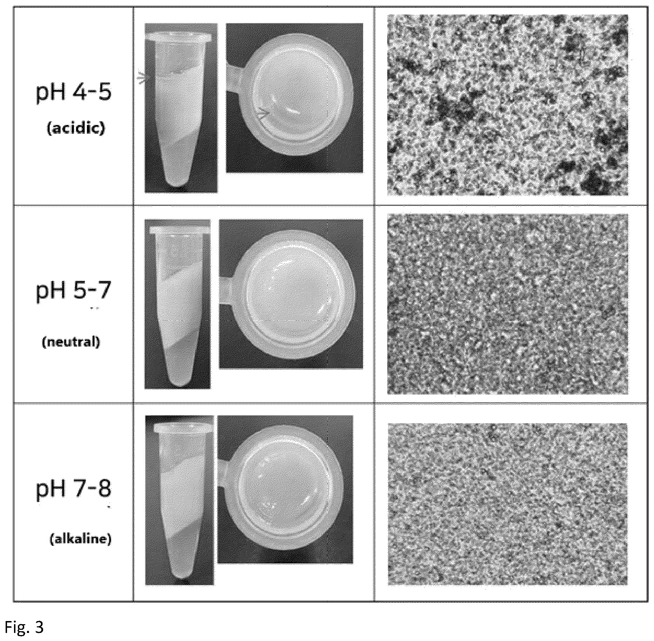

As can be seen from Table 9 and FIG. 3 below, the untreated group was stable without oil separation under all conditions (Comparative Example 1-11). The mixture treatment group exhibited noticeable oil separation under pH 4-5 (acidic) conditions, and was stable under pH 5-7 (neutral) and pH 7-8 (alkaline) conditions (Example 1-27). This indicates that the mixture did not maintain stability of the oleosome preparation under acidic conditions, but maintained stability of the oleosome preparation under neutral and alkaline conditions. In addition, the color of the oleosome preparation was maintained under neutral and alkaline conditions, but under acidic conditions, the color of the oleosome preparation itself slightly changed to yellow, thus meaning that the oleosome preparation was unstable. The particles of the oleosome preparation depending on pH under a microscope was observed. The result shows that the oleosome particles were evenly distributed and had fluidity under neutral and alkaline conditions, whereas the oleosome particles agglomerated and lacked fluidity, and the oil particles burst under acidic conditions. This shows that neutral and alkaline conditions are most suitable for maintaining the stability of oleosome preparations, when applying a mixture of 1,2-hexanediol and 2-methyl-1,3-propanediol to oleosome preparations.

**[Table 9]**

| | Composition | Centrifugation time | | | | |
|---|---|---|---|---|---|---|
| pH conditions | | 5 min | 10 min | 15 min | 20 min | 30 min |
| pH 4-5 (acidic) | Comparative Example 1-11 | +++ | +++ | +++ | +++ | +++ |
| | Example 1-27 | +++ | +++ | + | + | -- |
| pH 5-7 (neutral) | Comparative Example 1-11 | +++ | +++ | +++ | +++ | +++ |
| | Example 1-27 | +++ | +++ | +++ | ++ | + |
| pH 7-8 (alkali) | Comparative Example 1-11 | +++ | +++ | +++ | +++ | +++ |
| | Example 1-27 | +++ | +++ | +++ | ++ | + |

### Experimental Example 3. Determination of antimicrobial contamination effect of oleosome preparation composition

In order to determine the antimicrobial contamination effect of the compound used for oleosome treatment, the detection of bacteria left in oleosomes was determined 7 days after the compound treatment. To determine the antimicrobial effect, oleosome treated with each compound was added to MLA (modified letheen agar) medium, a bacterial culture medium, and SDA (Sabouraud's dextrose agar) medium, which is a fungal culture medium, at different concentrations, and solidified in petri dishes to prepare solid media and then the growth of cultured bacteria and fungi was observed. The bacteria were cultured at 3°C for 48 hours, fungi were cultured at 30°C for 72 hours, and then the detection of bacteria was observed to determine the composition and concentration that inhibited bacterial growth. The bacteria may be *B. subtilis, P. aeruginosa, E. coli,* and *Staphylococcus aureus,* and the fungi may be *Candida albicans* and A. *niger.*

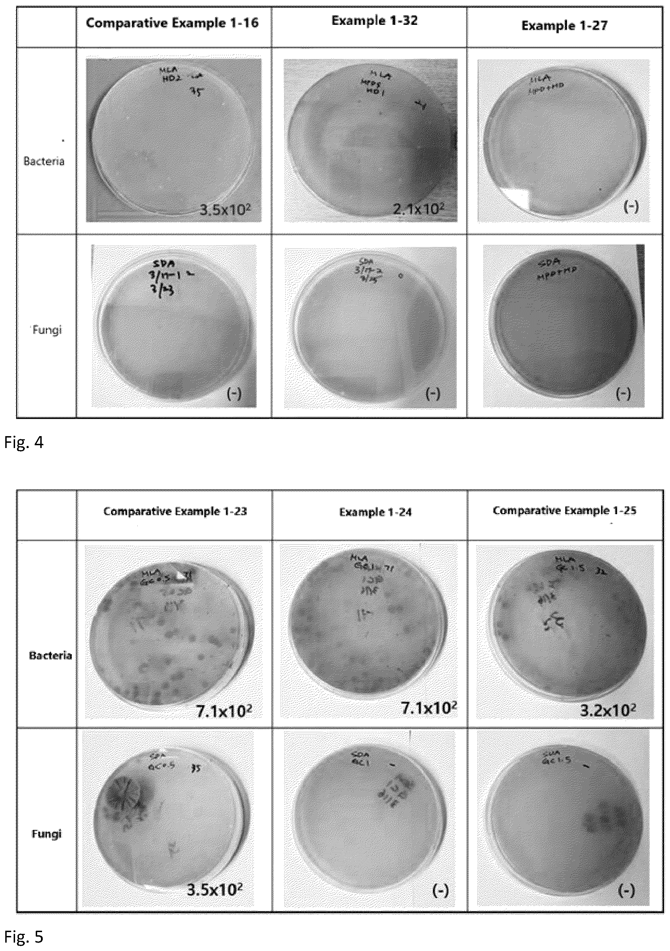

As can be seen from Table 10 and FIG. 4 below, both bacteria and fungi were detected in the oleosomes treated with 1,2-hexanediol and 2-methyl-1,3-propanediol alone (Comparative Examples 1-17 to 1-18, Example 1-23, Example 1-31). When the oleosomes were treated with 2% 1,2-hexanediol alone, the growth of bacteria was inhibited by more than 90% and the growth of fungi was inhibited by 100% (Comparative Example 1-16), but the oleosome had a side effect of making oleosome preparation unstable and was thus inapplicable. However, when oleosomes were treated with 2% of 1,2-hexanediol and 2-methyl-1,3-propanediol (1:5), the growth of both bacteria and fungi was inhibited by 100%, thus exhibiting a preservative effect and the oleosome preparation was also stable (Example 1-27). Accordingly, it can be seen that the composition has the effects of preventing bacterial contamination of oleosomes and inhibiting bacterial growth to maintain the excellent stability of the oleosome preparation.

**[Table 10]**

| Conditions | Bacteria | Fungi |
|---|---|---|
| Comparative Example 1-16 | ++ | - |
| Comparative Example 1-17 | +++ | +++ |
| Comparative Example 1-18 | +++ | +++ |
| Example 1-23 | +++ | +++ |
| Example 1-31 | +++ | +++ |
| Example 1-27 | - | - |
| Example 1-32 | ++ | + |
| Example 1-33 | +++ | +++ |

| | | |
|---|---|---|
| In Table 10, whether or not microbial growth was inhibited is indicated as follows. - : No growth, + : <10² detected, ++: <10³ detected, +++: >10³ detected | | |

### Experimental Example 4. Antimicrobial boosting effect of mixture of 1,2-hexanediol and 2-methyl-1,3-propanediol

The antimicrobial boosting effect was determined when oleosomes were treated with a mixture of 1,2-hexanediol and 2-methyl-1,3-propanediol in combination with other antimicrobial agents.

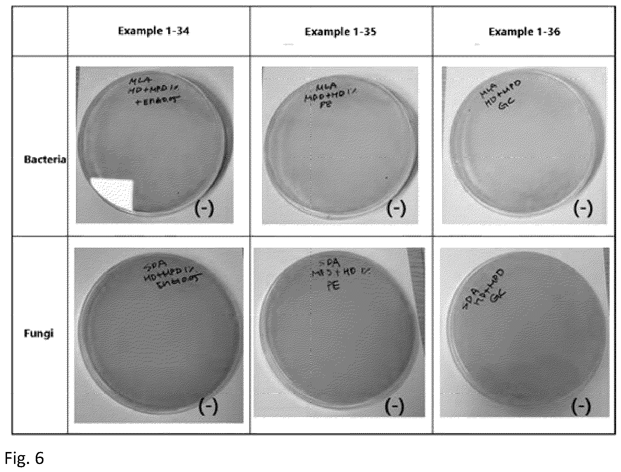

As can be seen from Table 11 below and FIGs. 5 to 7, among the glyceryl ether compounds commonly used in cosmetics, ethylhexylglycerin (hereinafter abbreviated as "EHG"), among the glyceryl ester compounds, glyceryl caprylate (hereinafter abbreviated as "GC"), and phenoxyethanol (hereinafter abbreviated as "PE") as other preservative were tested. When oleosomes were treated with a mixture of these compounds alone, the preservative effect was somewhat reduced even at high contents (Comparative Examples 1-19 to 1-25). However, when oleosomes were treated with a lower concentration of each compound with a mixture of 1% 1,2-hexanediol and 5% 2-methyl-1,3-propanediol, growth of both bacteria and fungi was inhibited by 100%, which indicates that the combination treatment has the preservative effect. In addition, when oleosomes were treated only with 2% or less of a mixture of 1,2-hexanediol and 2-methyl-1,3-propanediol (1:5), the antiseptic effect was slightly reduced (Example 1-32), but when oleosomes were treated with the mixture in combination of each other compound, the antiseptic effect was improved at 1%. This indicates that the mixture of 1,2-hexanediol and 2-methyl-1,3-propanediol had an excellent effect of boosting the antiseptic effect of other compounds (Examples 1-34 to 1-36).

**[Table 11]**

| | Bacteria | Fungi |
|---|---|---|
| Comparative Example 1-19 | +++ | ++ |
| Comparative Example 1-20 | +++ | ++ |
| Comparative Example 1-21 | ++ | - |
| Comparative Example 1-22 | ++ | - |
| Comparative Example 1-23 | ++ | ++ |
| Comparative Example 1-24 | ++ | - |
| Comparative Example 1-25 | ++ | - |
| Example 1-32 | ++ | - |
| Example 1-34 | - | - |
| Example 1-35 | - | - |
| Example 1-36 | - | - |

| | | |
|---|---|---|
| In Table 11, whether or not microbial growth is inhibited is indicated as follows. - : No growth, + : <10² detected, ++: <10³ detected, +++: >10³ detected | | |

As described above, the present inventors conducted tests on various compounds to find a mixture that stably maintains the oleosome preparation and has a preservative effect. Among the linear diol-based, branched diol-based, glyceryl ester/ether compounds, and other preservatives, commonly used compounds such as 1,2-hexanediol, 2-methyl-1,3-propanediol, ethylhexylglycerin, glyceryl caprylate, and phenoxyethanol were selected and the preparation stability was tested and the antimicrobial contamination effect thereof was determined.

First, stability of commonly used diol compounds was tested. The result shows that linear diol compounds, glyceryl ester/ether compounds, and other preservatives destabilized the oleosome preparations, thus causing oil leakage. However, branched diol compounds effectively maintained the stability of the oleosome preparation. 1,2-propanediol, 1,3-propanediol, 1,3-butanediol, and 1,2-hexanediol, were identified as linear diol compounds, ethylhexylglycerin and glyceryl caprylate were identified as glyceryl ester/ether compounds, phenoxyethanol was identified as other preservative, and 2-methyl-1,3-propanediol, 2,2-dimethyl-1,3-propanediol, 2-methyl-2-propyl-1,3-propanediol, and 3-methyl-1,3-butanediol were identified as branched diol compounds. In terms of structural characteristics, the linear structure weakens the unique structure of the oleosome, which is a spherical structure surrounded by monolayer phospholipids, thereby causing oil leakage, but the branched structure makes the oleosome structure stronger, thus stabilizing the preparation and preventing oil leakage.

In addition, it was found that linear diol compounds, glyceryl ester/ether compounds, and other preservatives, when used alone, break down the oleosome preparations, but when used in combination with branched diol compounds and applied to the oleosome preparation, stably maintained the oleosome preparation. Thus, it was found that branched diol compounds have the effect of preventing linear diol compounds, glyceryl ester/ether compounds, and other preservatives from breaking down the oleosome preparations.

A combination of 2-methyl-1,3-propanediol with 1,2-hexanediol, ethylhexylglycerin, glyceryl caprylate, and phenoxyethanol was tested on the oleosome preparation. The result shows that the oleosome preparation was stably maintained and caused no oil leakage. When each of 1,2-hexanediol, ethylhexylglycerin, glyceryl caprylate, or phenoxyethanol was applied directly to the oleosome preparation, the oleosome preparation was broken and oil leakage occurred. However, when 1,2-hexanediol, ethylhexylglycerin, glyceryl caprylate, and phenoxyethanol were dispersed in 2-methyl-1,3-propanediol and stabilized, and then the oleosome preparation was treated with the resulting dispersion, the oleosome preparation was stabilized and oil leakage did not occur.

In addition, the antimicrobial contamination effect was determined. The result shows that treatment with 1,2-hexanediol or 2-methyl-1,3-propanediol alone resulted in low antimicrobial contamination effect, whereas treatment with a mixture of 1,2-hexanediol and 2-methyl-1,3-propanediol made the oleosome preparation stable and resulted in much better antimicrobial contamination effect.

In addition, oleosomes were treated with other compounds (ethylhexylglycerin, phenoxyethanol, glyceryl caprylate), which caused a problem of insufficient preservative effect and thus required large amounts when used alone, were used in combination with a mixture of 1,2-hexanediol and 2-methyl-1,3-propanediol. The result shows that the antimicrobial contamination effect was excellent even in spite of a smaller amount, and the oleosome preparation was also maintained stably. This indicates the preservative boosting effect of the mixture of 1,2-hexanediol and methylpropanediol was also determined.

### Preparation Example

UV protection emulsion preparation including oleosome preparation
Phase 1 oil phase
Stabilized oleosome (oleosome 88%, 1,2-hexanediol 2%, 2-methyl-1,3-propanediol 10%) 12.0%
Octyl methoxycinnamate 1.0%
Octocrylene 0.5%
Ethylhexyl salicylate 0.5%
Benzophenone-3 0.5%
Titanium dioxide 2.0%
Novemer EC-1 1.1%
Phase 2 aqueous phase
Urea 5.0%
Polyglycerin-10 5.0%
Water to 100%
Phase 3
Fragrance 0.14%
Preservative 0.4%

### Treatment process

1. Phase 1 ingredients were added along with low shear mixing.
2. Phase 2 ingredients were added together.
3. Phase 2 was slowly added to phase 1 with low shear mixing.
4. Phase 3 was added and stirred.

Although the preferred embodiments of the present invention have been disclosed for illustrative purposes, those skilled in the art will appreciate that various modifications, additions and substitutions are possible, without departing from the scope and spirit of the invention as disclosed in the accompanying claims. Accordingly, the embodiments disclosed herein are provided only for better understanding of the present invention and should not be construed as limiting the spirit and scope of the present invention. The scope of protection of the present invention should be interpreted by the following claims and all techniques within the equivalent scope should be interpreted as falling within the scope of the present invention.

### [Industrial Applicability]

According to the present invention, oleosomes that exhibit improved preparation stability, effectively inhibit bacterial growth, and prevent microbial contamination to provide have excellent preservative effects, and are thus useful in cosmetics and the like.

## Claims

1. A method of preparing an oleosome preparation composition having excellent stability and antibacterial properties comprising:
1) preparing an oleosome;
2) treating the oleosome with a mixture of a C1 to C5 branched diol compound, a C1 to C10 linear diol compound, and a glyceryl ether/ester compound or other preservative; and
3) preparing an oleosome having a pH of 5.0 to 8.0,
wherein the branched diol compound comprises at least one selected from the group consisting of 2-methyl-1,3-propanediol, 2,2-dimethyl-1,3-propanediol, 2-methyl-2-propyl-1,3-propanediol, and 3-methyl-1,3-butanediol,
the linear diol compound comprises at least one selected from the group consisting of 1,3-propanediol, 1,3-butanediol, 1,2-hexanediol, and 1,2-octanediol,
the glyceryl ether/ester compound comprises at least one selected from the group consisting of ethylhexylglycerin, glyceryl caprylate, glyceryl stearate, glyceryl undecylenate, caprylyl glyceryl ether, and glyceryl laurate, and
the other preservative is phenoxyethanol.

2. The method according to claim 1, wherein the branched diol compound is 2-methyl-1,3-propanediol, the linear diol compound is 1,2-hexanediol, the glyceryl ether compound is ethylhexylglycerin, the glyceryl ester compound is glyceryl caprylate, and the other preservative is phenoxyethanol.

3. The method according to claim 1, wherein step 1) comprises:
soaking seeds in water;
washing the seeds;
wet-grinding the washed seeds with water at a weight ratio of 1:2;
filtering the resulting seeds to obtain a ground solution;
mixing the ground solution with sodium bicarbonate; and
centrifuging the resulting mixture to obtain oleosomes.

4. The method according to claim 3, wherein the seeds are plant seeds including safflower seeds, hempseeds, sunflower seeds, camellia seeds, citron seeds, or rapeseed seeds.

5. An oleosome preparation composition having excellent stability and antibacterial properties prepared using the method according to any one of claims 1 to 4.

6. A cosmetic product comprising the oleosome preparation composition having excellent stability and antibacterial properties according to claim 5.

7. The cosmetic product according to claim 6, wherein the cosmetic product is selected from the group consisting of skin care products, makeup products, hair care products, sunscreens, body care products, hand sanitizers, deodorants, exfoliating products, topical products, dermatological products, and acne care products.

8. An oleosome preparation composition having excellent stability and antibacterial properties comprising:
79.3 to 94.5 parts by weight of oleosome;
0.5 to 4.0 parts by weight of 1,2-hexanediol;
0.01 to 0.1 parts by weight of ethylhexylglycerin;
0.1 to 1 part by weight of glyceryl caprylate; and
4.8 to 20 weight parts of 2-methyl-1,3-propanediol.

9. The oleosome preparation composition according to claim 8, wherein the oleosome preparation composition has a pH of 5.0 to 8.0.

10. The oleosome preparation composition according to claim 8, wherein the oleosome preparation composition is formulated as a pack, an ointment, a lotion, a solubilization, a suspension, an emulsion, a cream, a gel, a spray, a papule, a plaster, a patch, or a pain relief patch.
